(19) Europäisches Patentamt / European Patent Office / Office européen des brevets

(11) **EP 3 368 919 B1**

(12) **FASCICULE DE BREVET EUROPEEN**

(45) Date de publication et mention de la délivrance du brevet:
**23.12.2020 Bulletin 2020/52**

(21) Numéro de dépôt: **16787821.4**

(22) Date de dépôt: **25.10.2016**

(51) Int Cl.:
*A61B 6/12* (2006.01)    *A61B 6/06* (2006.01)
*A61B 6/03* (2006.01)    *G21K 1/04* (2006.01)
*G01T 7/00* (2006.01)    *A61B 6/00* (2006.01)

(86) Numéro de dépôt international:
**PCT/EP2016/075698**

(87) Numéro de publication internationale:
**WO 2017/072125 (04.05.2017 Gazette 2017/18)**

(54) **COLLIMATEUR TOURNANT POUR DETERMINER LA POSITION D'UN ELEMENT MUNI DE CAPTEURS DANS UN SYSTEME D'IMAGERIE PAR RAYONS X**

DREH-KOLLIMATOR ZUR BESTIMMUNG DER LAGE EINES MIT DETEKTOREN AUSGESTATTETEN ELEMENTES IN EINEM RÖNTGEN ABBILDUNGSSYSTEM

ROTATING COLLIMATOR FOR DETERMINING THE POSITION OF AN ELEMENT EQUIPPED WITH DETECTORS IN A X-RAY IMAGING SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.10.2015 FR 1560194**

(43) Date de publication de la demande:
**05.09.2018 Bulletin 2018/36**

(73) Titulaires:
• **Surgiqual Institute**
  **38700 La Tronche (FR)**
• **Université Grenoble Alpes**
  **38400 Saint-Martin-d'Hères (FR)**
• **Centre Hospitalier Universitaire Grenoble**
  **38700 La Tronche (FR)**

(72) Inventeurs:
• **GRONDIN, Yannick**
  **73800 Arbin (FR)**
• **AUGERAT, Philippe**
  **38330 St Ismier (FR)**
• **CINQUIN, Philippe**
  **38330 Saint Nazaire les Eymes (FR)**
• **DESBAT, Laurent**
  **38000 Grenoble (FR)**
• **DECROUEZ, Marion**
  **73000 Chambéry (FR)**

(74) Mandataire: **Novagraaf Technologies**
  **Bâtiment O2**
  **2, rue Sarah Bernhardt**
  **CS90017**
  **92665 Asnières-sur-Seine Cedex (FR)**

(56) Documents cités:
**WO-A1-01/37287    US-A1- 2008 118 023**

**Description**

**DOMAINE TECHNIQUE ET ART ANTÉRIEUR**

La présente invention a trait au domaine de l'imagerie par rayons X, et concerne plus particulièrement celui de la localisation spatiale d'éléments dans un système d'imagerie par rayons X.

Elle s'applique notamment à l'imagerie dite interventionnelle lors de laquelle, on cherche à déterminer précisément le positionnement d'un outil en même temps que l'on réalise une acquisition d'images, par exemple lors d'une intervention chirurgicale ou d'une opération réalisée à l'aide de cet outil.

Elle s'applique également à la calibration d'un système d'imagerie par rayons X, et permet de déterminer la position d'un imageur par rapport à un référentiel donné ou à une source de rayons X.

Le document FR 2 841 118 présente un système d'imagerie par rayon X comprenant un moyen de détermination du positionnement d'un appareil de radiographie à l'aide d'une mire fixée sur un objet dont on acquiert une image.

Dans un tel système l'image de la mire interfère avec celle de l'objet que l'on souhaite étudier.

Le document US 6 490 475 présente quant à lui un système de fluoroscopie dans lequel on détermine le positionnement d'éléments du système à l'aide de marqueurs. Un traitement numérique est alors effectué pour pouvoir retirer ensuite l'empreinte de ces marqueurs de l'image finale.

Le document US 7 065 393 concerne un procédé de calibration d'un système d'imagerie par rayonnement X à l'aide de capteurs inertiels disposés sur différents éléments du système.

Ce type de capteur peut être soumis à des perturbations et poser des problèmes de fiabilité.

Il se pose le problème de trouver un nouveau dispositif permettant de déterminer de manière fiable la position d'un élément muni de capteurs dans un système d'imagerie par rayons X.

**EXPOSÉ DE L'INVENTION**

Selon un aspect, la présente invention concerne un dispositif pour l'imagerie par rayons X comprenant un collimateur formé d'un support, ledit support étant une plaque sous forme de disque ou de portion de disque, le support étant apte à être mobile en rotation sur lui-même et par rapport à un axe de rotation, le support comprenant une région opaque aux rayons X, cette région opaque étant traversée par au moins une première fente et au moins une deuxième fente, la première fente et la deuxième fente étant transparentes aux rayons X, la première fente et la deuxième fente s'étendant chacune dans un plan donné parallèle au plan principal du support et respectivement dans une première direction passant par l'axe de rotation et dans deuxième direction qui n'est pas sécante à l'axe de rotation du collimateur.

A l'aide d'un tel dispositif on peut déterminer de manière fiable la position dans un référentiel donné d'un élément muni de capteurs de rayons X destinés à recevoir des faisceaux de rayons X issus des fentes du collimateur.

Un tel collimateur permet d'estimer de manière précise la position dudit élément tout en limitant la dose de rayons X reçue.

Avantageusement, la première fente et la deuxième fente ont des largeurs différentes. Cela peut permettre à un dispositif de détection d'établir une distinction entre un faisceau de rayons X issu de la première fente et un faisceau de rayons X issu de la deuxième fente.

Un autre moyen d'établir une telle distinction est de prévoir des fentes avec des compositions différentes. La première fente peut être alors constituée d'un premier matériau radio-transparent, tandis que la deuxième fente est constituée d'un deuxième matériau radio-transparent.

Avantageusement, le support est doté en outre d'une région transparente aux rayons X située dans le plan donné et juxtaposée à ladite région.

Avec un tel agencement du collimateur, on peut successivement masquer puis dévoiler une source de rayons X devant lequel ce collimateur est disposé et mis en rotation, ce qui permet d'alterner une phase d'imagerie et une phase dans laquelle on se sert du collimateur pour déterminer une position d'un élément de détection.

Selon un autre aspect, un mode de réalisation de la présente invention prévoit un système d'imagerie par rayons X comprenant :

- une source de rayons X,
- un moyen de détermination de la position d'au moins un élément par rapport à un référentiel donné, l'élément étant muni d'un ou plusieurs capteurs de rayons X, le moyen de détermination comprenant :

    - un dispositif tel que défini plus haut,
    - une unité de traitement configurée pour déterminer au moins une droite d'intersection entre un premier faisceau de rayons X et un deuxième faisceau de rayons X détectés par un même capteur de rayons X parmi lesdits capteurs, le premier faisceau étant issu de la source de rayons X et ayant traversé la première fente et le deuxième faisceau de rayons X étant issu de la source de rayons X et ayant traversé la deuxième fente.

Le système d'imagerie est de préférence muni d'un imageur. Lorsque le support du collimateur est doté d'une région radio-transparente aux rayons X juxtaposée à la région radio-opaque, le système peut être agencé de sorte que, lors d'une rotation du collimateur sur lui-même, la source est pendant une première phase dévoilée à un imageur par la région transparente puis pendant une deuxième phase masquée partiellement à cet imageur par la région opaque et dévoilée uniquement par l'intermédiaire de la première fente et de la deuxième fente.

Dans ce cas, l'unité de traitement peut être synchronisée avec le collimateur de sorte que :

- lors de ladite première phase l'unité de traitement déclenche l'acquisition d'au moins une image radiographique par l'imageur,
- lors de ladite deuxième phase l'unité de traitement estime la position dudit élément.

On peut ainsi produire une image radiographique pendant la première phase et réaliser une navigation dudit élément ou une calibration du système d'imagerie lors de la deuxième phase.

Selon une possibilité de mise en œuvre, l'élément dont on détermine la position est un imageur doté de capteurs de rayons X, de préférence d'au moins 4 capteurs de rayons X.

Ces capteurs peuvent être des pixels de l'imageur ou des capteurs spécifiques disposés sur une matrice de pixels de l'imageur.

Dans ce cas, le moyen de détermination est configuré en particulier pour déterminer la position de l'imageur par rapport à la source de rayons X.

Selon une autre possibilité de mise en œuvre, l'élément dont on détermine la position est un objet déplaçable par rapport à la source de rayons X, tel qu'un outil chirurgical, auquel le ou les capteurs de rayons X est ou sont rattaché(s).

Dans ce cas, le système d'imagerie par rayons X peut comprendre en outre :

- un imageur apte à produire une image radiographique et
- un dispositif d'affichage pour afficher une image radiographique produite par l'imageur et rendant compte d'un déplacement de l'objet.

Selon une possibilité de mise en œuvre le système d'imagerie peut être de type à arceau (« C-arm » selon la terminologie anglo-saxonne).

Un mode de réalisation prévoit un collimateur déplaçable par rapport à la source de rayons X hors de son axe de rotation. Dans ce cas, on peut prévoir un collimateur avec uniquement une région opaque traversée par des fentes radio-transparentes. Une fois qu'une position de l'élément a été détectée, pour permettre de réaliser une acquisition d'image, le collimateur peut être déplacé et retiré du chemin entre la source de rayons X et un imageur.

Selon un autre aspect, un mode de réalisation de la présente invention concerne un procédé de calibration d'un système d'imagerie par rayons X tel que défini plus haut et dans lequel ledit élément est un imageur muni d'au moins 4 capteurs de rayons X. Ce procédé comprend une détermination de la position de l'imageur par rapport à la source ou par rapport à un référentiel de référence lié à une salle dans laquelle se situe le système d'imagerie, cette détermination de la position de l'imageur comprenant une estimation par une unité de traitement couplée à l'imageur d'au moins 4 droites distinctes reliant chacune la source et un capteur parmi lesdits au moins 4 capteurs de l'imageur.

## BRÈVE DESCRIPTION DES DESSINS

La présente invention sera mieux comprise à la lecture de la description d'exemples de réalisation donnés, à titre purement indicatif et nullement limitatif, en faisant référence aux dessins annexés sur lesquels :

- la figure 1 illustre un exemple de collimateur tournant doté d'une région radio-opaque traversée par des fentes radio-transparentes, le collimateur étant destiné à être intégré à un système d'imagerie par rayons X pour permettre de déterminer la position d'un élément détecteur de rayons X au sein du système,
- les figures 2A-2B illustrent un exemple particulier d'agencement des fentes radio-transparentes dans l'épaisseur de la région radio-opaque du collimateur,
- la figure 3 illustre un exemple de dispositif d'entrainement en rotation du collimateur,
- les figures 4A-4B illustrent différentes position d'un système d'imagerie par rayons X doté du collimateur tournant et configuré pour réaliser alternativement une détermination de la position d'un détecteur de rayons X et une production d'image radiographique d'un objet cible,
- la figure 5 illustre de manière schématique une modélisation d'un système source collimateur détecteur,
- la figure 6 illustre de manière schématique une première méthode d'estimation du déplacement d'un imageur dans un système d'imagerie muni d'un collimateur tournant,
- la figure 7 illustre de manière schématique une deuxième méthode d'estimation du déplacement d'un imageur dans un système d'imagerie muni d'un collimateur tournant,
- la figure 8 illustre un système d'imagerie C-arm dans lequel un collimateur tournant est intégré,
- la figure 9 illustre un système d'imagerie par rayons X à collimateur tournant permettant de suivre la navigation d'un objet muni d'au moins un capteur de rayons X,
- la figure 10 illustre une variante d'agencement du collimateur tournant,

Des parties identiques, similaires ou équivalentes des différentes figures portent les mêmes références numériques de façon à faciliter le passage d'une figure à l'autre.

Les différentes parties représentées sur les figures ne le sont pas nécessairement selon une échelle uniforme, pour rendre les figures plus lisibles.

## EXPOSÉ DÉTAILLÉ DE MODES DE RÉALISATION PARTICULIERS

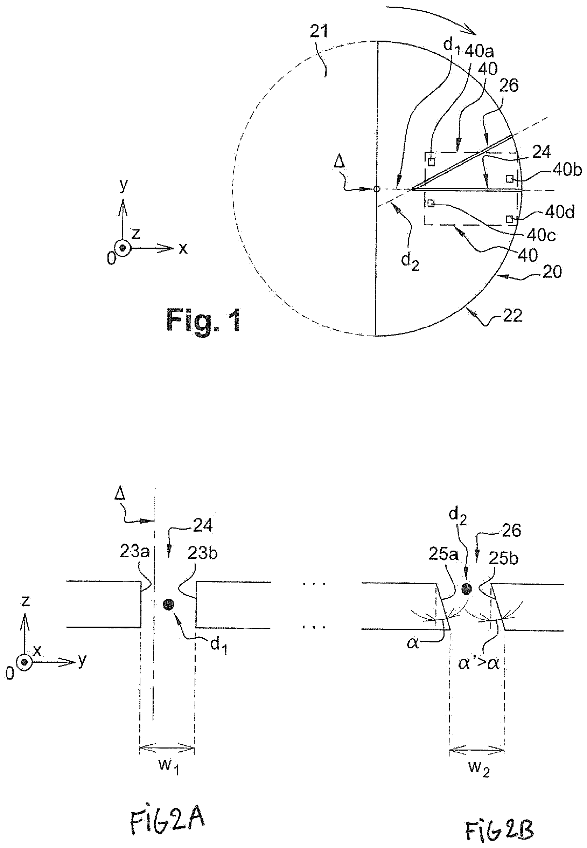

On se réfère tout d'abord à la figure 1 sur laquelle un exemple de cible tournante encore appelée collimateur est représenté.

Le collimateur est utilisé pour déterminer le positionnement dans un référentiel donné d'au moins un élément apte à détecter des rayons X. Cet élément peut être par exemple un détecteur 40 de rayons X muni de capteurs 40a, 40b, 40c, 40d situés dans un même plan.

Le collimateur est ici représenté selon une vue de dessus et formé d'un support 20 apte à être mis en rotation sur lui-même devant une source de rayons X. La source de rayons X (non représentée) est par exemple sous forme d'un tube émetteur de rayons X. L'axe de rotation $\Delta$ du support 20 passe par une normale au plan principal du support 20 c'est-à-dire un plan qui passe par le support 20 et qui est parallèle au plan [O ; x ; y] donné sur la figure 1. Le collimateur 20 est destiné à être disposé à une distance prédéterminée et connue de la source de rayons X.

Dans cet exemple de réalisation, le support 20 est une plaque, sous forme d'un disque ou d'une portion de disque, avec une région radio-opaque 22 en un matériau ayant un pouvoir important d'absorption vis-à-vis des rayons X tel que par exemple le plomb ou le tungstène. La plaque peut avoir une épaisseur (mesurée parallèlement à l'axe z d'un repère [O ; x ; y ; z] donné sur la figure 1) de l'ordre de plusieurs millimètres. La région radio-opaque 22 du support 20 est traversée par deux fentes 24, 26 transparentes aux rayons X. Les fentes 24, 26 peuvent être vides ou remplies d'un matériau ayant une faible absorption vis-à-vis des rayons X, tel que par exemple du carbone. Les fentes 24, 26 ont une largeur (mesurée dans le plan [O;x;y] du repère [O ; x ; y ; z]) qui peut être par exemple inférieure à 1 mm.

Les deux fentes 24, 26 radio-transparentes sont agencées sur le support 20 de sorte que dans un plan parallèle au plan principal du support 20, une première fente 24 s'étend dans une première direction $d_1$ qui est sécante à l'axe de rotation $\Delta$ du support 20, tandis qu'une deuxième fente 26 s'étend quant à elle dans une deuxième direction $d_2$ qui ne passe pas par l'axe de de rotation $\Delta$ du support 20. Dans cet exemple de réalisation particulier, les directions $d_1$ et $d_2$ des fentes 24, 26 ne sont pas colinéaires et réalisent un angle non-nul l'une par rapport à l'autre.

Avec un tel agencement du collimateur, lorsque le support 20 est en rotation sur lui-même par rapport à l'axe de rotation $\Delta$, il est possible pour un capteur de rayons X de détecter un premier faisceau de rayons X issu de la première fente 24 et un deuxième faisceau de rayons X issu de la deuxième fente 26.

Une différence de largeur entre les fentes 24, 26 peut être prévue afin d'établir une distinction entre le premier faisceau et le deuxième faisceau, la largeur (dimension mesurée parallèlement au plan [O ; x; y] des fentes influant sur la largeur temporelle de signaux reçu par les capteurs 40a, 40b, 40c, 40d.

Ainsi, lorsque la deuxième fente 26 est prévue par exemple avec une largeur $W_2$ supérieure à celle $W_1$ de la première fente, du fait de la réception d'un faisceau ayant traversé la première fente on capte un premier signal de largeur temporelle moins importante qu'un deuxième signal induit par un deuxième faisceau ayant traversé la deuxième fente, le premier et le deuxième signal ayant des amplitudes sensiblement égales.

Un autre moyen d'établir une distinction entre un premier faisceau issu de la première fente et un deuxième faisceau issu de la deuxième fente est de prévoir celles-ci avec des compositions différentes. Par exemple, la première fente 24 est sous forme d'un trou est ainsi constituée d'air tandis que la deuxième fente est remplie d'un matériau radio-transparent par exemple un matériau plastique d'une épaisseur prédéterminée. L'amplitude d'un signal ayant traversé la première fente et détectée par le capteur peut être différente de celle de la fente uniquement constituée d'air si l'on considère une épaisseur de matériau plastique suffisante.

Pour tenir compte de la divergence du faisceau de rayons X issu de la source de rayons X, la première fente 24, et la deuxième fente 26 peuvent être prévues avec des sections transversales différentes. Par section « transversale » on entend une section prise dans un plan orthogonal au plan principal du support 20, autrement dit dans un plan qui est parallèle au vecteur z du repère [O ; x ; y ; z] sur la figure 1.

Des vues en coupe transversale de la première fente 24 et de la deuxième fente 26 sont données respectivement sur les figures 2A et 2B.

La première fente 24, i.e. celle située sur un rayon du disque et qui coupe l'axe de rotation Δ a une section transversale délimitée entre une zone 23a de la région radio-opaque 22 qui est parallèle à une normale au plan principal du support 22 et à l'axe de rotation Δ et une autre zone 23b. La section transversale peut être rectangulaire. Dans ce cas l'autre zone 23b est parallèle à une normale au plan principal du support 22. Pour tenir compte de la divergence du faisceau de rayons X, l'autre zone 23b peut en variante réaliser un angle non-nul avec la normale au plan principal du support 22.

La deuxième fente 26, i.e. celle qui n'est pas située sur un rayon du disque et qui ne coupe pas l'axe de rotation Δ a une section transversale définie entre des zones 25a, 25b qui sont inclinées et réalisent respectivement un angle $\alpha$ et un angle $\alpha'$ (avec $\alpha' > \alpha > 0$) par rapport au plan principal du support 20 ou à l'axe de rotation Δ. L'inclinaison des zones 25a, 25b est prévue en fonction de la divergence du faisceau de rayons X émanant de la source de rayons X.

Les fentes 24, 26 balaient sensiblement le même champ spatial. Pour que l'intégralité de la surface du détecteur 40 soit balayée par des rayons X issus de la source 10 et traversant les fentes 24, 26, on peut prévoir des fentes de longueur par exemple supérieure à 4 cm, dans le cas par exemple où l'on considère un détecteur 40 de dimension maximale de 40 cm et un facteur d'agrandissement du faisceau de rayons X de l'ordre de 10 entre le collimateur 20 et le détecteur 40.

Le système source de rayons X/collimateur permet de produire deux faisceaux de rayons X tournants, chaque faisceau traversant une fente 24 ou 26 pouvant être assimilé à un plan de rayons X. Le système source-collimateur permet ainsi de générer deux plans de rayons X tournants que les capteurs de rayon X du détecteur 40 sont aptes à détecter.

La détection de ces plans par un capteur de rayons X donné du détecteur 40 permet de déduire une droite d'intersection entre ces deux plans reliant un capteur à la source de rayon X. Si l'on considère par exemple 4 capteurs 40a, 40b, 40c, 40d différents du détecteur 40 on peut prévoir une détection de 4 droites différentes d'intersection. Par détection des plans sécants de rayons X on peut déterminer la position du détecteur 40 de rayons X, en particulier par rapport à la source 10.

Un mode de réalisation de l'invention prévoit ainsi un moyen de détermination de la position du détecteur 40 par détection de faisceaux ou plans de rayons X sécants issus du collimateur.

Le positionnement des fentes 24, 26 sur le support 20 et en particulier la position et l'orientation de la première fente 24 par rapport à la deuxième fente 26 est connu de ce moyen de détermination, de même que la vitesse de rotation du support 20.

Ainsi, le positionnement l'un par rapport à l'autre des deux faisceaux ou plans de rayons X traversant les fentes 24, 26 est connu. L'intervalle de temps entre un instant où un capteur de rayons X est parcouru par le premier faisceau ou plan de rayons X traversant la première fente et un instant où ce même capteur de rayons X est parcouru par le deuxième faisceau ou plan de rayons X traversant la première fente est également connu puisqu'il dépend de la vitesse de rotation du support 20.

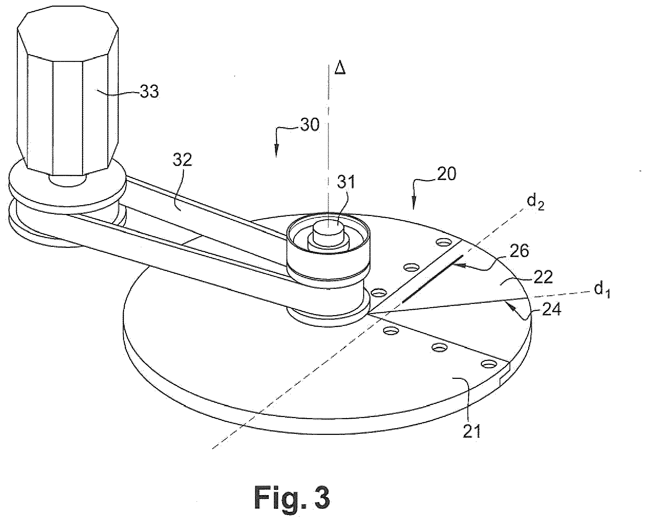

La mise en rotation du support 20 du collimateur est quant à elle assurée par un dispositif d'entrainement dont un exemple de réalisation est donné de manière schématique sur la figure 3. Dans cet exemple particulier, le support est sous forme d'un disque lié en son centre à un arbre 31 entrainé en rotation par l'intermédiaire d'une courroie 32 elle-même reliée à un autre arbre 33.

Un mode de réalisation particulier prévoit un collimateur amovible et/ou qui peut être déplacé au sein du système d'imagerie. Ainsi, un dispositif (non représenté) permettant de déplacer le collimateur 20 par rapport à la source 10 et de le retirer du champ de vision du détecteur 40 peut être prévu.

Avantageusement, le support 20 du collimateur comprend une région transparente aux rayons X, soit sous forme d'une portion vide autour de la région opaque 22 du support 20 soit sous forme d'une région du support 20 en matériau radio-transparent qui est co-planaire avec la région radio-opaque 22.

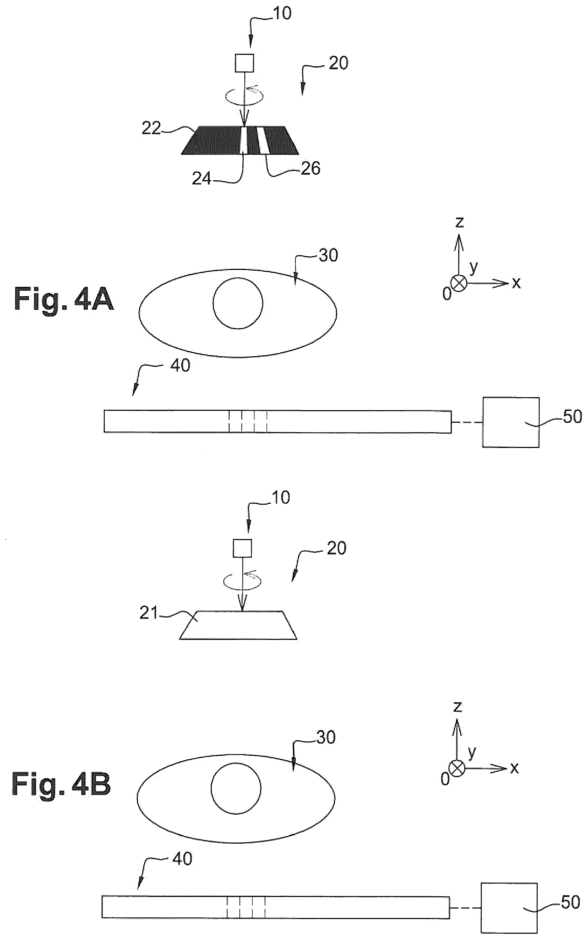

Sur les figures 4A-4B, on a représenté un système d'acquisition d'images radiologiques doté d'un collimateur tel que décrit précédemment.

L'agencement du collimateur avec une région opaque 22 et une région transparente 21 permet, lorsque celui-ci est mis en rotation sur lui-même d'alterner, une première phase dite de localisation ou de calibration, pendant laquelle on détermine la position d'un élément détecteur de rayons X, par exemple du détecteur 40 et une deuxième phase d'acquisition d'image lors de laquelle une image radiologique d'un objet cible 30 est générée. Le système d'imagerie peut être appliqué à l'imagerie médicale, l'objet cible 30 considéré étant dans ce cas un patient.

Sur la figure 4A, le collimateur se trouve dans une position correspondant à la phase de localisation, le détecteur 40 vu de la source 40 étant masqué par la région radio-opaque 22 du collimateur. Les dimensions du collimateur ainsi que sa position par rapport à la source 10 et au détecteur 40 sont prévues de telles sorte que lors de la phase de localisation, le faisceau de rayons X issu de la source 10 et masqué par la région radio-opaque 22 n'est susceptible d'atteindre le détecteur 40 que par le biais des fentes 24, 26.

Le détecteur 40 est dans cet exemple sous forme d'un imageur ou d'une matrice de capteurs de rayons X répartis sur un même plan. La détection des plans de rayons X issus des fentes du collimateur peut être réalisée soit par des capteurs intégrés aux pixels de la matrice ou de l'imageur, soit par des capteurs spécifiques disposés sur la matrice de pixels. Un exemple de capteur de rayons X comprend un scintillateur de type cristal de CsI convertissant les rayons X en lumière UV ou visible, le cristal étant disposé sur un transistorTFT (pour « Thin Film Transistor ») sensible à la lumière. Un autre exemple de capteur intègre un matériau semi-conducteur, tel que le Sélénium amorphe, apte à réaliser conversion directe des rayons X en charges électriques. Les capteurs de rayons X utilisés peuvent avoir par exemple une forme carrée de côté par exemple de l'ordre de 1 mm. Les capteurs utilisés ont de préférence une réponse temporelle inférieure à 0.1 ms.

Sur la figure 4B, le collimateur se trouve dans une position correspondant à la phase d'acquisition d'image, le détecteur 40 vu de la source 40 étant visible à travers la région radio-transparente 21 du collimateur. L'agencement du collimateur est tel que lors de la phase d'acquisition d'image, le faisceau de rayons X issu de la source 10 traverse la région radio-transparente 21. Dans ce cas, aucune zone radio-opaque du collimateur ne vient perturber l'image radiologique de l'objet cible 30.

Ainsi, pour un même tour du collimateur sur lui-même dont la durée peut être par exemple de l'ordre de 200 ms, on passe successivement d'une phase de localisation à une phase d'acquisition d'image et on peut ainsi successivement déterminer la position du détecteur 40 tout en limitant l'irradiation, puis produire une image radiographique sans perturber ou encombrer le champ de vision de l'imageur.

Pour permettre de déterminer la position du détecteur 40 par dans un référentiel donné, par exemple par rapport à la source de rayons X ou à un point fixe donné, à l'aide d'une détection de plans de rayons X issus des fentes 24, 26 du collimateur, le détecteur 40 peut être couplé à une unité de traitement 50 intégrée ou reliée au détecteur.

L'unité de traitement 50 numérique peut être dotée d'au moins un processeur, d'au moins un module mémoire et d'au moins un périphérique d'entrée. Le processeur peut être intégré au détecteur 40 et par exemple sous forme d'un microprocesseur, ou d'un circuit FPGA ou bien être connecté au détecteur 40 et par exemple sous forme d'un processeur d'unité centrale ou de station de travail. Un réseau de processeurs peut être également mis en œuvre. Le module mémoire peut comprendre, par exemple une mémoire morte ROM, une mémoire programme EPROM, une mémoire vive dynamique DRAM ou tout autre type de mémoire volatile et/ou non volatile. Des algorithmes sous forme d'instructions peuvent être stockés dans la mémoire programme, et permettent d'effectuer des étapes de traitement numérique notamment pour déterminer la position du détecteur 40 à partir de signaux issus de capteurs de rayons X et produire des images de l'objet cible 30. Un programme, permettant de mettre en œuvre de tels traitements peut être enregistré dans le module mémoire de l'unité 50 de traitement ou tout support mémoire lisible par l'unité 50 de traitement.

L'unité 50 de traitement est également configurée pour permettre de synchroniser les phases d'acquisition d'image radiologique et de localisation avec la rotation du collimateur. En particulier, la phase d'acquisition d'image, lors de laquelle une image est produite est mise en œuvre dans un intervalle de temps au cours duquel la région radio-transparente dévoile la source 10 au détecteur 40. La phase de localisation, lors de laquelle une estimation de la position d'un élément détecteur de rayon X est réalisée, est effectuée dans un intervalle de temps au cours duquel la région radio-opaque masque partiellement la source 10 au détecteur 40, celle-ci n'étant visible que par le biais des fentes 24, 26. Pour réaliser une telle synchronisation l'unité de traitement 50 dispose d'une information relative à la vitesse de rotation du collimateur.

La phase de localisation permet de déterminer la position du détecteur 40.

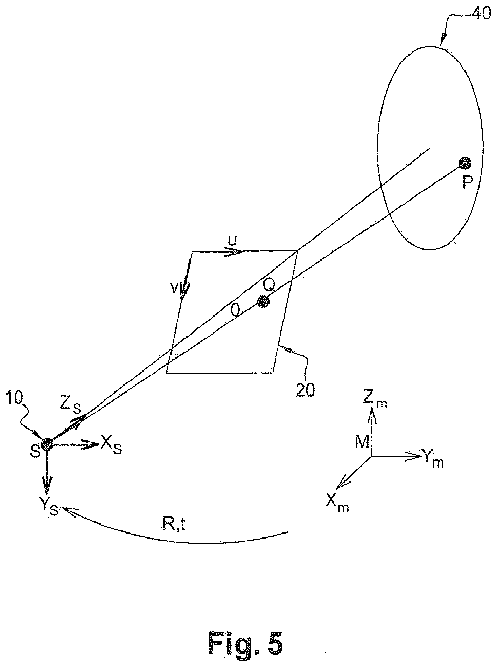

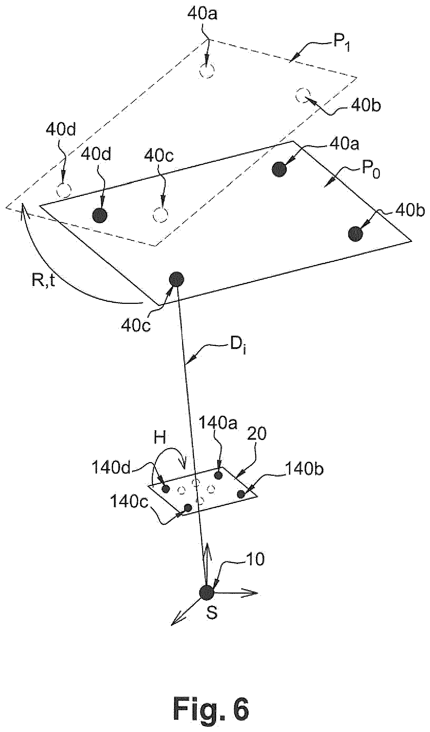

Un exemple de méthode de détermination de la position du détecteur 40 par rapport à un référentiel donné, par exemple par rapport à la source 10, va à présent être décrit en liaison avec les figures 5 et 6.

On se réfère à présent à la figure 5 sur laquelle le système source-collimateur-détecteur est représenté de manière schématique.

Pour effectuer une modélisation de ce système, l'unité de traitement 50 peut utiliser différents référentiels, dont un référentiel appelé référentiel monde dont le centre est un point M et dans lequel les coordonnées d'un point P sont exprimés par exemple en mètre.

Un référentiel source dont le centre S est la source 10 de rayonnement X est également défini. Le référentiel source est doté d'un premier axe Xs parallèle à un vecteur u, d'un deuxième axe YS parallèle à un autre vecteur v. Un troisième axe du référentiel source est quant à lui normal au plan (u,v) défini par les vecteurs u et v.

Ce plan (u, v) également appelé « plan image » ou « plan collimateur» correspond ici au plan principal du support 20 du collimateur dans lequel se trouvent les fentes 24, 26. On considère ici un référentiel correspondant au plan du collimateur 20. Ce référentiel permet d'exprimer les coordonnées des éléments d'intérêts du système projectif.

Les coordonnées d'un point P dans le référentiel source peuvent être exprimées par exemple en mètre.

Un point O appelé le « point principal » correspond à une projection orthogonale du centre S sur le plan du support 20 du collimateur. La distance SO est notée f et est appelée « distance focale ». O a pour coordonnées $(u_0, v_0)$ dans le référentiel image, la droite SO est appelée « axe principal ».

La projection d'un point P du détecteur 40 sur le plan collimateur (u, v) au point Q est exprimée par une combinaison de transformations géométriques:

Les coordonnées du point P sont exprimées dans le référentiel monde (M, Xm, Ym, Zm) et sont converties dans le référentiel de la source (S, Xs, Ys, Zs) : ce sont des paramètres extrinsèques. P est ensuite projeté sur le plan (u,v) du collimateur au point Q en fonction des caractéristiques internes du système source-détecteur : ce sont les paramètres intrinsèques.

Notons les coordonnées de P
dans le référentiel monde : (X,Y,Z)
dans le référentiel source : (Xs,Ys, Zs)
On a la relation :

$$\begin{bmatrix} X_s \\ Y_s \\ Z_s \end{bmatrix} = R \begin{bmatrix} X \\ Y \\ Z \end{bmatrix} + t = [R|t] \begin{bmatrix} X \\ Y \\ Z \end{bmatrix}$$

R,t sont respectivement une opération de rotation et de translation qui permettent de passer du référentiel monde au référentiel source. L'opération de rotation R peut s'exprimer comme le produit de 3 matrices de rotations, chacune de ses 3 matrices représentant une rotation autour d'un axe donné du référentiel.

Q est le projeté de P dans le plan du collimateur et a pour coordonnées (x,y,z) dans le référentiel source :

$$\begin{bmatrix} x \\ y \\ z \end{bmatrix} = \begin{bmatrix} f/Z_s & 0 & 0 \\ 0 & f/Z_s & 0 \\ 0 & 0 & f/Z_s \end{bmatrix} \begin{bmatrix} X_s \\ Y_s \\ Z_s \end{bmatrix}$$

Cette expression est obtenue par la relation de Thalès. Q a pour coordonnées (u,v) dans le plan du collimateur et on a la relation :

$$\begin{bmatrix} u \\ v \end{bmatrix} = \begin{bmatrix} k_u & 0 & u_0 \\ 0 & k_v & v_0 \end{bmatrix} \begin{bmatrix} x \\ y \\ 1 \end{bmatrix}$$

ku et kv sont communément des facteurs de conversion de mètres en pixels exprimés en pixel/m. Dans notre cas, on reste dans un système métrique que l'on soit dans le plan du détecteur 40 ou le plan du collimateur 20, on a donc ku=kv=k=1.

Par conséquent, on obtient la relation suivante entre Q (u,v) et P(Xs,Ys, Zs) :

$$\begin{bmatrix} au \\ av \\ a \end{bmatrix} = \begin{bmatrix} f & 0 & u_0 \\ 0 & f & v_0 \\ 0 & 0 & 1 \end{bmatrix} \begin{bmatrix} X_s \\ Y_s \\ Z_s \end{bmatrix} = K \begin{bmatrix} X_s \\ Y_s \\ Z_s \end{bmatrix}$$

K est une matrice représentant les paramètres intrinsèques du système source-détecteur : f, u0 et v0. f est la distance séparant la source et le collimateur 20 qui est connue de l'unité de traitement 50, u0 et v0 étant les coordonnées cartésiennes (en mètre) du point principal dans le plan du collimateur 20.

La relation entre un point P exprimé dans le référentiel monde et son projeté Q dans le plan (u, v) est :

$$Q = K[R|t]P$$

Une calibration d'un système d'imagerie tel que décrit précédemment peut être réalisée en usine, de sorte que lorsque l'on utilise ensuite ce système, l'unité de traitement 50 connait au moins une position calibrée, appelée « position de référence » du détecteur 40 relativement à la source 10. Ainsi, des positions respectives de référence de plusieurs capteurs de rayons X 40a, 40b, 40c, 40d du détecteur 40 par rapport à la source 10 sont connues. La position du collimateur 20 relativement à la source 10 est également connue de l'unité de traitement 50.

Lorsque le système d'imagerie est en fonctionnement, le détecteur 40 plan n'est plus nécessairement dans sa position de référence et peut se trouver dans une nouvelle position que l'on souhaite déterminer. La position du détecteur 40 par rapport à la source 10 peut être destinée à varier, même pendant que l'on cherche à obtenir des images de l'objet cible 30.

Un mouvement du détecteur 40, et de plusieurs de ses capteurs 40a, 40b, 40c, 40d de rayons X d'une position $P_0$, par exemple la position de référence vers une nouvelle position $P_1$ est représenté de manière schématique sur la figure 6.

D'une manière générale, chaque nouvelle position du détecteur 40 et donc d'un capteur de ce dernier est le résultat d'une rotation R par rapport au référentiel lié à la source 10 et d'une translation t de la position de référence.

L'unité de traitement 50 est configurée pour estimer ces opérateurs R, t afin de déterminer chaque nouvelle position du détecteur plan 40.

Pour déterminer une nouvelle position $P_1$ du détecteur 40, l'unité de traitement 50 est configurée pour calculer une homographie H permettant de passer des projections 140a, 140b, 140c, 140d respectives des capteurs 40a, 40b, 40c, 40d données sur le plan du collimateur 20 lorsque le détecteur 40 se trouve dans une première position $P_0$ vers les projections 140a, 140b, 140c, 140d respectives des capteurs 40a, 40b, 40c, 40d données sur le plan du collimateur 20 lorsque le détecteur 40 se trouve dans cette nouvelle position $P_1$.

Une projection d'un capteur du détecteur 40 dans le plan du collimateur 20, correspond à l'intersection d'une droite reliant la source 10 et ce capteur avec le plan principal du support 20, cette droite étant elle-même l'intersection des deux plans de rayons X issus des fentes 24, 26 du collimateur.

Pour réaliser le calcul de l'homographie H, l'unité 50 de traitement effectue un appariement des projections des capteurs 40a, 40b, 40c, 40d de rayons X sur deux séries de mesures.

Cet appariement peut être réalisé en partant de l'hypothèse que le déplacement du détecteur 40 plan est faible, en particulier inférieur à plusieurs mm ou degrés. Ainsi, un critère proximité des projetés est utilisé pour effectuer les appariements.

Ainsi, lorsqu'on cherche à apparier un projeté avec un autre projeté, c'est le projeté plus proche qui est considéré.

Les capteurs 40a, 40b, 40c, 40c du détecteur 40 utilisés pour déterminer la position du détecteur 40 sont de préférence éloignés les uns des autres afin d'éviter toute erreur d'appariement. Par exemple, lorsqu'on considère un détecteur de forme rectangulaire, les capteurs 40a, 40b, 40c, 40c sont répartis de préférence à proximité de chaque sommet du rectangle formé par le détecteur.

On peut partir de l'hypothèse que la matrice de paramètres intrinsèques K est inchangée dans les deux positions du détecteur 40 plan dans la mesure où le collimateur 20 est solidaire de la source 10 ou que la distance source collimateur 20 est fixe.

Il existe alors une relation reliant tous les opérateurs et la matrice de paramètres intrinsèques K:

$$HK = K(R - t\vec{n}/d)$$

Avec $\vec{n}$ la normale du détecteur 40 dans la position $P_0$ de référence, et d, sa distance par rapport au référentiel source. La matrice K est connue et l'homographie H est calculée.

HK peut être décomposé en 3 matrices : la matrice K, la matrice de rotation R par rapport au référentiel lié à S, et une matrice représentant le produit $t\vec{n}/d$, duquel on déduit t.

Pour que cette méthode garantisse l'obtention de la matrice de rotation R, et la matrice de translation t de manière univoque, on utilise de préférence au moins 4 capteurs de rayons X du détecteur 40 ou disposés sur le détecteur 40. Afin d'améliorer la précision et la rapidité de détermination la position du détecteur 40, on peut utiliser des mesures provenant d'un nombre de capteurs supérieur à 4.

En variante de la méthode décrite précédemment, la détermination de la position du détecteur 40 par rapport à un référentiel donné et en particulier par rapport à la source 10 peut être réalisée à l'aide d'une méthode de type appelée P3P issue de méthodes décrites dans les documents « Complété solution classification for the perspective-three-point problem », de Gao, et al. (2003), IEEE Transactions on Pattern Analysis and Machine Intelligence, 25(8), 930-943 et « Method for registration of 3D shapes », de Besl et al. (1992), In Robotics-DL tentative pp. 586-606, International Society for Optics and Photonics.

On considère un référentiel (Xs, Ys, Zs) dont le centre S est la source 10 de rayons X, un plan (xi, yi) du collimateur 20, des point U, V, W, situés dans le plan du collimateur 20 ayant pour projetés respectivement les points A, B, C dans les plan du détecteur 40, et qui correspondent respectivement aux emplacements de capteurs $40_1$, $40_2$ et $40_3$, sur le détecteur 40. On considère également le point D dans le plan du détecteur correspondant à un autre capteur

$40_4$ de rayons X. On considère par ailleurs les angles $\varphi_{u,w} \varphi_{v,w} \varphi_{u,v}$ correspondant respectivement à $\widehat{SUW}$ , $\widehat{SVW}$ ,

$\widehat{SUV}$.

Le détecteur 40 est un dispositif planaire dont on souhaite déterminer la position et l'orientation, en évaluant les distances SA, SB, SC, SD.

Ainsi, au moins 4 points A, B, C et D, et donc au moins 4 capteurs de rayons X $40_1$, $40_2$, $40_3$, $40_4$ sont ici utilisés. Comme pour l'autre méthode décrite précédemment en liaison avec la figure 6, un nombre de capteurs supérieur à 4 peut être prévu pour améliorer la précision de l'estimation de la position du détecteur 40.

Les capteurs $40_1$, $40_2$, $40_3$, $40_4$ correspondant aux points A, B, C, D sont situés chacun sur une droite d'intersection entre deux plans de rayons X ayant traversé les deux fentes du collimateur et ayant détecté un signal correspondant à cette intersection.

Les calculs mis alors en œuvre par l'unité de traitement 50 sont basés sur la formule d'Al-Kashi suivante : $AB^2 = SB^2 + SA^2 - 2SASB\cos\varphi_{u,v}$
Pour les 3 points A, B et C, non-colinéaires, on a :

$$SB^2 + SC^2 - 2SBSC\cos\varphi_{v,w} - BC^2 = 0$$

$$SA^2 + SC^2 - 2SASC\cos\varphi_{u,w} - AC^2 = 0$$

$$SA^2 + SB^2 - 2SASB\cos\varphi_{u,v} - AB^2 = 0$$

Si on divise par $SC^2$ et que l'on note y=SB/SC et x=SA/SC, ce système d'équation s'écrit :

$$y^2 + 1 - 2y\cos\varphi_{v,w} - BC^2/SC^2 = 0$$

$$x^2 + 1 - 2x\cos\varphi_{u,w} - AC^2/SC^2 = 0$$

$$x^2 + y^2 - 2xy\cos\varphi_{u,v} - AB^2/SC^2 = 0$$

On note $m=AB^2/SC^2$ et $am=BC^2/SC^2$ et $bm=AC^2/SC^2$, on obtient le système :

$$y^2 + 1 - 2y\cos\varphi_{v,w} - am = 0$$

$$x^2 + 1 - 2x\cos\varphi_{u,w} - bm = 0$$

$$x^2 + y^2 - 2xy\cos\varphi_{u,v} - m = 0$$

On remplace $m = x^2 + y^2 - 2xy\cos\varphi u$, dans les deux premières équations, ce qui donne le système :

$$(1-a)y^2 - ax^2 - (2\cos\varphi_{v,w})y + (2a\cos\varphi_{u,v})xy + 1 = 0$$

$$(1-b)x^2 - by^2 - (2\cos\varphi_{u,w})x + (2a\cos\varphi_{u,v})xy + 1 = 0$$

Les positions des capteurs $40_1$, $40_2$, $40_3$, $40_4$, les uns par rapport aux autres sur le détecteur 40 sont connues, ce qui permet autrement dit de connaître AB, BC, AC.

La position du collimateur 20 par rapport à la source 10 est connue, ce qui permet de connaître SU, SV, SW ainsi que les angles $\varphi_u$, $\varphi_{u,w}$, $\varphi_{v,w}$.

En résolvant le système d'équations ci-dessus 4 solutions possibles des distances SA, SB, SC et 4 valeurs possibles des vecteurs SA, SB et SC exprimés dans le référentiel lié à la source peuvent être déduites par l'unité de traitement 50. Le point D correspondant au $4^{ème}$ capteur $40_4$ de rayons X dans le plan du collimateur 20, permet à l'unité de traitement 50 de déterminer sans ambiguïté la position et l'orientation réelle du détecteur 40.

Une localisation du détecteur 40 avec une précision de localisation inférieure à la dimension d'un capteur peut être obtenue à l'aide de l'une ou l'autre des méthodes décrites précédemment.

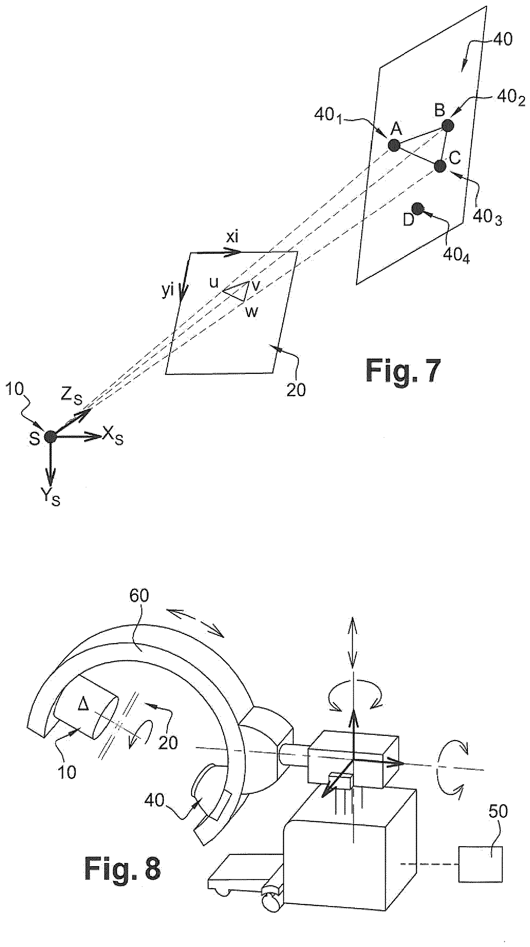

Selon un exemple particulier d'application, un collimateur tournant tel que décrit précédemment peut être intégré à un système d'imagerie par rayons X de type à arceau (« C-arm » selon la terminologie anglo-saxonne) tel que celui représenté par exemple sur la figure 8 dans lequel la source 10 de rayons X est reliée à un détecteur 40 de rayons X par l'intermédiaire d'un bras 60 en arceau ou en forme de C.

La source 10 et le détecteur 40 sont destinés à se déplacer, et en particulier à tourner autour d'un objet cible, ici un patient, pour réaliser des projections selon plusieurs angles de vue. Ces projections sont ensuite exploitées par l'unité de traitement 50 couplée au détecteur 40. L'unité de traitement 50 est dans ce cas configurée en outre pour mettre en œuvre un algorithme de reconstruction afin d'obtenir une image 3D ou volumique, représentant plusieurs coupes de l'objet cible imagé. Dans ce type de système employé par exemple dans des blocs opératoires, des informations de positionnement relatif de la source 10 et du détecteur 40 peuvent être utilisées afin de construire des images tomographiques 3D.

En effet, dans les données d'entrée de l'algorithme de reconstruction utilisées par l'unité de traitement 50 d'image pour produire les images 3D figurent la position du dispositif C-arm relativement à l'objet imagé ainsi que la position relative de la source 10 et du détecteur 40. Or d'éventuelles imperfections mécaniques ainsi que l'effet de la gravité peuvent avoir tendance à modifier la distance entre la source 10 et le détecteur 40. L'unité de traitement 50 peut être configurée de sorte à réaliser une succession d'estimations de la position du détecteur 40 afin de produire des images radiographiques, en particulier dynamiques, de précision accrue. Ces estimations peuvent permettre d'effectuer une calibration du système d'imagerie en temps réel pendant qu'il est en fonctionnement et que des images radiographiques sont produites. Ce type de calibration également appelée calibration « on-line », peut être réalisé par exemple lors d'une opération chirurgicale.

Lorsque le système d'imagerie précédemment décrit est utilisé pour réaliser de la tomographie, en particulier de type CBCT (pour « Cone Beam Computed Tomography »), chaque image produite par le biais de l'imageur peut être associée à des paramètres intrinsèques de calibration comprenant une information de positionnement du détecteur et qui sont utilisés par des algorithmes de reconstruction d'image afin de produire une image sans artefact.

Outre ce type de calibration appelé « intrinsèque », le dispositif à collimateur peut permettre de réaliser une calibration « extrinsèque »,

La calibration extrinsèque consiste à déterminer les positions du système d'imagerie dans un référentiel de référence lié au lieu où se trouve le système d'imagerie et l'objet cible, par exemple un référentiel lié à une salle d'opération où se trouve un patient dans le cas d'une application à l'imagerie médicale lors d'une chirurgie.

Une telle calibration est utilisée pour effectuer une reconstruction d'images tomographiques (3D), pour effectuer une navigation d'un outil chirurgical, ou un recalage d'image, par exemple avec des images tomodensitométriques (scanner) acquises antérieurement.

La calibration extrinsèque comprend une estimation de la position du détecteur relativement à la source par une méthode telle que décrite précédemment, ainsi qu'une détermination de la position de la source 10 dans le référentiel de référence. La position de la source 10 peut être déterminée par un dispositif de localisation connu de l'Homme du Métier, par exemple un dispositif optique tel que décrit dans le document US 5°923°727, ou par une centrale inertielle de mesure telle que décrite dans le document : « A low-cost tracked C-arm (TC-arm) upgrade system for versatile quantitative intraoperative imaging », de Amiri, et al., (2013). International Journal of Computer Assisted Radiology and Surgery, 1-17).

Pour un système d'imagerie à arceau statique, utilisable par exemple pour réaliser une coronarographie, dans lequel la source 10 et le détecteur 40 ne sont pas mobiles, un autre type de calibration appelée « off-line » peut être mis en œuvre.

Lors de ce type de calibration, on détermine les positions relatives de la source de rayons X, et du détecteur 40 à l'aide d'un dispositif à collimateur tel que décrit précédemment avant d'utiliser le système pour produire des images radiographiques.

Une variante de réalisation du collimateur décrit précédemment prévoit un support avec une surface cette fois entièrement radio-opaque et traversée de deux fentes radio-transparentes.

Dans ce cas, le collimateur est utilisé principalement pour mettre en œuvre une phase de localisation lors de laquelle on estime la position d'un ou plusieurs capteurs de rayons X ou d'un détecteur de rayons X, en détectant l'intersection des plans de rayons X issus des fentes. Lors de cette phase de localisation, le collimateur 20 est ainsi placé dans le parcours de rayons X issus de la source 10 de rayons X.

Puis, lorsque cette phase de localisation est terminée, et par exemple qu'une calibration du système d'imagerie a été effectuée, le collimateur peut être ensuite déplacé par rapport à la source de rayons X de sorte qu'il ne se trouve plus sur son axe de rotation et dans le parcours de rayons X entre la source et l'imageur. On peut alors effectuer une acquisition d'image radiologique d'un objet cible ou d'un patient par l'imageur sans que le collimateur ne gêne cette acquisition.

Le passage d'une position de localisation ou de calibration à une position d'acquisition du système d'imagerie par déplacement du collimateur est assuré par un dispositif de déplacement.

Avec un tel agencement du collimateur, la dose qu'un patient est susceptible de recevoir lors d'une phase de localisation ou de calibration est très faible et par exemple 100 fois inférieure à la dose à laquelle il peut être exposé lors d'une phase d'acquisition d'image réalisée sans collimateur.

Un système d'imagerie doté d'un collimateur tel que décrit précédemment peut être mis en œuvre pour réaliser une navigation et connaître la position et/ou la trajectoire d'un élément mobile. Par « élément » on entend ici aussi bien une partie de l'anatomie d'un patient qu'un objet ou un outil amené à être déplacé par rapport à la source de rayons X.

Dans le cas où l'on souhaite réaliser une navigation d'un outil le ou les capteurs de rayons X utilisé(s) pour détecter les plans de rayons X issus des fentes du collimateur, sont disposés directement sur l'outil.

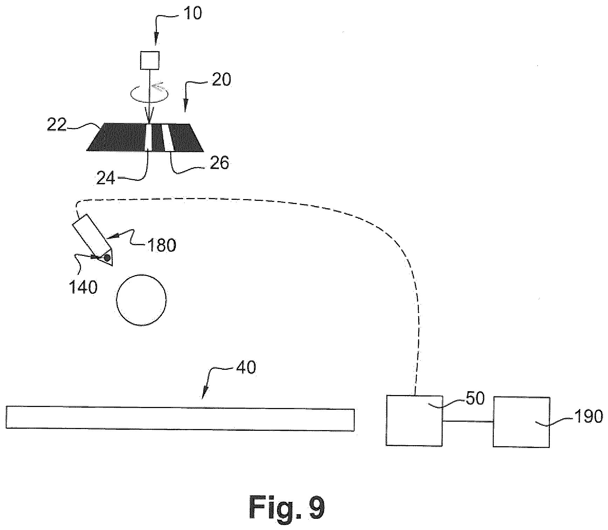

Sur la figure 9, l'objet dont on souhaite suivre la trajectoire et la position par rapport à un référentiel donné est par exemple un instrument chirurgical 180 muni d'un capteur de rayons X 140 fixé à proximité ou sur la pointe de l'instrument 180 et destiné à se déplacer entre la source 10 de rayons X et le détecteur 40 de rayons X sous forme d'imageur. Le capteur 140 de rayons X est couplé à une unité de traitement 50 telle qu'évoquée précédemment.

Le collimateur 20 tournant est dans ce cas utilisé pour permettre de déterminer la position de l'instrument 180 et de suivre sa trajectoire.

La distance entre la source 10 de rayons X et l'imageur 40 de rayons X est cette fois connue et fixe, et l'on détermine la position du capteur 140 fixé sur l'instrument 180 par exemple de la même manière que dans l'exemple de réalisation décrit précédemment pour déterminer la position d'un capteur de l'imageur.

Le capteur 140 sur l'instrument 180 permet de détecter une droite d'intersection entre un premier plan de rayons X issu de la première fente du collimateur 20 et un deuxième plan de rayons X issu de la deuxième fente du collimateur 20.

On déduit de cette droite une projection du capteur 140 dans le plan de l'imageur, cette projection correspondant à une intersection de la droite d'intersection avec le plan de l'imageur.

On peut ensuite suivre le déplacement de l'instrument 180 en suivant le déplacement de cette projection dans le plan de l'imageur 40. Un dispositif d'affichage 190 d'image radiographique permettant de suivre en temps réel ce déplacement peut être prévu. Un modèle numérique de l'objet 180 est pour cela créé, puis affiché sur le dispositif d'affichage 190. Le modèle numérique est alors animé dans une image en fonction du déplacement que fait l'objet 180.

Un tel principe de navigation s'applique au suivi en temps réel d'opérations chirurgicales, mais aussi à certains examens ou pour le positionnement précis de patients par rapport à un appareil d'imagerie par rayons X.

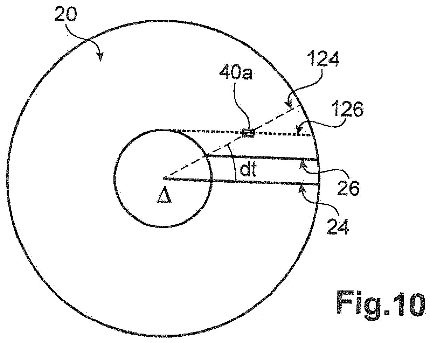

Une variante d'agencement du collimateur tournant est illustrée sur la figure 10, sur laquelle les deux fentes 24, 26 du collimateur sont représentées selon une position du collimateur prise à un premier instant $t_0$.

Là encore, les fentes 24, 26 sont agencées de sorte que la première fente 24 s'étend dans une première direction $d_1$ qui est sécante à l'axe de rotation $\Delta$ du support 20 tandis que la deuxième fente 26 s'étend quant à elle dans une deuxième direction $d_2$ qui ne passe pas par l'axe de rotation $\Delta$ du support 20. L'agencement du collimateur diffère de celui donné précédemment en liaison avec la figure 1, en ce que les directions $d_1$ et $d_2$ des fentes 24, 26 sont cette fois parallèles.

Un capteur 40a de rayons X est susceptible de détecter un plan 126 de rayons X issu de la deuxième fente 26 lorsque celle-ci se trouve dans une position donnée au premier instant $t_0$, et de détecter un autre plan 126 de rayons X issu de la première fente 24 lorsque celle-ci se trouve dans une position correspondant à un instant ultérieur $t_0+dt$, l'intersection entre ces deux plans 124, 126 renseignant sur la position du capteur 40a.

## Revendications

**1.** Dispositif pour l'imagerie par rayons X comprenant :

un collimateur formé d'un support (20), ledit support étant une plaque sous forme de disque ou de portion de disque,
le support étant apte à être mobile en rotation sur lui-même et par rapport à un axe (A) de rotation traversant un plan du support appelé « plan principal du support »,
le support comprenant une région opaque (22) aux rayons X, cette région opaque étant traversée par au moins une première fente (23) et au moins une deuxième fente (24), la première fente et la deuxième fente étant transparentes aux rayons X, la première fente et la deuxième fente s'étendant chacune dans un plan donné parallèle au plan principal du support et respectivement dans une première direction passant par l'axe de rotation et dans une deuxième direction qui n'est pas sécante à l'axe de rotation, de sorte que, lorsqu'une source de rayon X est positionnée pour fournir des rayons X, deux plans de rayons X tournants sont générés en aval desdites fentes.

2.  Dispositif selon la revendication 1, dans lequel la première fente et la deuxième fente ont des largeurs différentes.

3.  Dispositif selon l'une des revendications 1 ou 2, dans lequel le support (20) comprend en outre une région transparente (21) aux rayons X et située dans le plan donné et juxtaposée à la région opaque (22).

4.  Dispositif selon l'une des revendications 1 à 3, dans lequel la première direction et la deuxième direction réalisent entre elles un angle non-nul.

5.  Système d'imagerie par rayons X comprenant :

    - une source (10) de rayons X,
    - un moyen de détermination de la position d'au moins un élément (40, 180) par rapport à un référentiel donné, l'élément étant muni d'un ou plusieurs capteurs (40a, 40b, 40c, 40d, 140) de rayons X, le moyen de détermination comprenant :
    - un dispositif selon l'une des revendications 1 ou 2,
    - une unité de traitement (50) configurée pour déterminer au moins une droite d'intersection entre un premier faisceau de rayons X et un deuxième faisceau de rayons X détectés par un même capteur de rayons X parmi lesdits capteurs, le premier faisceau étant issu de la source de rayons X et ayant traversé la première fente et le deuxième faisceau de rayons X étant issu de la source de rayons X et ayant traversé la deuxième fente.

6.  Système d'imagerie par rayons X selon la revendication 5, comprenant un imageur et dans lequel le support (20) du collimateur est doté d'une région radio-transparente (21) aux rayons X juxtaposée à la région radio-opaque, le système étant agencé de sorte que, lors d'une rotation du collimateur sur lui-même, la source est pendant une phase dévoilée à un imageur par la région transparente puis pendant une autre phase masquée à cet imageur par la région opaque et dévoilée uniquement par l'intermédiaire de la première fente et de la deuxième fente, le système étant **caractérisé en ce que** l'unité de traitement est synchronisée avec la rotation du collimateur (20) de sorte que :

    - lors de ladite phase l'unité de traitement déclenche l'acquisition d'au moins une image radiographique par l'imageur,
    - lors de ladite autre phase l'unité de traitement estime la position dudit élément.

7.  Système d'imagerie selon l'une des revendications 5 ou 6, dans lequel ledit élément est un imageur (40) et dans lequel lesdits capteurs de rayons X sont des pixels de l'imageur ou sont disposés sur une matrice de pixels de l'imageur.

8.  Système d'imagerie selon l'une des revendications 5 à 7, le moyen de détermination étant configuré pour déterminer la position de l'imageur par rapport à la source.

9.  Système d'imagerie par rayons X selon l'une des revendications 5 ou 6, dans lequel l'élément est un objet (180) déplaçable par rapport à la source de rayons X, tel qu'un outil chirurgical, auquel le ou les capteurs (140) de rayons X est ou sont rattaché(s).

10. Système d'imagerie par rayons X selon la revendication 9, le système comprenant en outre :

    - un imageur (40) apte à produire une image radiographique et
    - un dispositif d'affichage (160) pour afficher une image radiographique produite par l'imageur et rendant compte du déplacement de l'objet.

11. Système d'imagerie par rayons X selon l'une des revendications 5 à 10, dans lequel la source et l'imageur sont reliés par un arceau.

12. Système d'imagerie par rayons X selon l'une des revendications 5 à 11, dans lequel le collimateur (20) est déplaçable par rapport à la source hors de son axe de rotation.

**13.** Procédé de calibration d'un système d'imagerie par rayons X selon l'une des revendications 5 à 12, dans lequel ledit élément est un imageur (40) muni d'au moins 4 capteurs de rayons X, comprenant la détermination de la position de l'imageur par rapport à la source ou par rapport à un référentiel de référence lié à une salle dans laquelle se situe le système d'imagerie, la détermination de la position de l'imageur comprenant une estimation par une unité de traitement couplée à l'imageur d'au moins 4 droites distinctes reliant chacune la source et un capteur parmi lesdits au moins 4 capteurs de l'imageur.

**Patentansprüche**

**1.** Röntgenstrahlenabbildungsvorrichtung, die Folgendes beinhaltet:

einen Kollimator, der durch einen Träger (20) gebildet ist, wobei der Träger eine Platte in Form einer Scheibe oder eines Scheibenabschnitts ist,
wobei der Träger fähig ist, um sich selbst und mit Bezug auf eine Drehachse (A), die durch eine Ebene des Trägers, die als "Trägerhauptebene" bezeichnet wird, verläuft, drehbar zu sein,
wobei der Träger einen für die Röntgenstrahlen undurchlässigen Bereich (22) beinhaltet, wobei dieser undurchlässige Bereich von mindestens einem ersten Schlitz (23) und mindestens einem zweiten Schlitz (24) durchlaufen wird, wobei der erste Schlitz und der zweite Schlitz für die Röntgenstrahlen durchlässig sind, wobei sich der erste Schlitz und der zweite Schlitz beide in einer gegebenen, zu der Trägerhauptebene parallelen Ebene und jeweils in einer ersten Richtung, die durch die Drehachse verläuft, und in einer zweiten Richtung, die die Drehachse nicht schneidet, erstrecken, sodass, wenn eine Röntgenstrahlenquelle für die Bereitstellung von Strahlen positioniert wird, stromabwärts der Schlitze zwei rotierende Röntgenstrahlenebenen generiert werden.

**2.** Vorrichtung nach Anspruch 1, wobei der erste Schlitz und der zweite Schlitz unterschiedliche Breiten aufweisen.

**3.** Vorrichtung nach einem der Ansprüche 1 oder 2, wobei der Träger (20) ferner einen für die Röntgenstrahlen durchlässigen Bereich (21) beinhaltet, der sich in der gegebenen Ebene befindet und an den undurchlässigen Bereich (22) angrenzt.

**4.** Vorrichtung nach einem der Ansprüche 1 bis 3, wobei die erste Richtung und die zweite Richtung zwischen sich einen Winkel ungleich null bilden.

**5.** Röntgenstrahlenabbildungssystem, das Folgendes beinhaltet:

- eine Röntgenstrahlenquelle (10),
- ein Mittel zum Bestimmen der Lage mindestens eines Elements (40, 180) mit Bezug auf ein gegebenes Bezugssystem, wobei das Element mit einem oder mehreren Röntgenstrahlendetektoren (40a, 40b, 40c, 40d, 140) ausgerüstet ist, wobei das Bestimmungsmittel Folgendes beinhaltet:
- eine Vorrichtung nach einem der Ansprüche 1 oder 2,
- eine Verarbeitungseinheit (50), die dazu konfiguriert ist, mindestens eine Schnittgerade zwischen einem ersten Röntgenstrahlenbündel und einem zweiten Röntgenstrahlenbündel, die durch einen Röntgenstrahlendetektor der mehreren Detektoren detektiert werden, zu bestimmen, wobei das erste Bündel aus der Röntgenstrahlenquelle stammt und den ersten Schlitz durchlaufen hat und das zweite Röntgenstrahlenbündel aus der Röntgenstrahlenquelle stammt und den zweiten Schlitz durchlaufen hat.

**6.** Röntgenstrahlenabbildungssystem nach Anspruch 5, das eine Abbildungsvorrichtung beinhaltet, und wobei der Träger (20) des Kollimators über einen röntgenstrahlendurchlässigen Bereich (21) verfügt, der an den röntgenstrahlenundurchlässigen Bereich angrenzt, wobei das System so eingerichtet ist, dass bei einer Drehung des Kollimators um sich selbst die Quelle während einer Phase durch den durchlässigen Bereich für eine Abbildungsvorrichtung offengelegt ist und dann während einer anderen Phase durch den undurchlässigen Bereich für diese Abbildungsvorrichtung verborgen ist und nur mittels des ersten Schlitzes und des zweiten Schlitzes offengelegt ist, wobei das System **dadurch gekennzeichnet ist, dass** die Verarbeitungseinheit derart mit der Drehung des Kollimators (20) synchronisiert ist, dass:

- während der einen Phase die Verarbeitungseinheit die Erfassung mindestens eines radiographischen Bilds durch die Abbildungsvorrichtung auslöst,
- während der anderen Phase die Verarbeitungseinheit die Lage des Elements ermittelt.

7. Abbildungssystem nach einem der Ansprüche 5 oder 6, wobei das Element eine Abbildungsvorrichtung (40) ist und wobei die Röntgenstrahlendetektoren Pixel der Abbildungsvorrichtung sind oder auf einer Pixelmatrix der Abbildungsvorrichtung angeordnet sind.

8. Abbildungssystem nach einem der Ansprüche 5 bis 7, wobei das Bestimmungsmittel zum Bestimmen der Lage der Abbildungsvorrichtung mit Bezug auf die Quelle konfiguriert ist.

9. Röntgenstrahlenabbildungssystem nach einem der Ansprüche 5 oder 6, wobei das Element ein mit Bezug auf die Röntgenstrahlenquelle bewegbares Objekt (180), wie etwa ein chirurgisches Instrument, ist, an dem der oder die Röntgenstrahlendetektoren (140) befestigt sind.

10. Röntgenstrahlenabbildungssystem nach Anspruch 9, wobei das System ferner Folgendes beinhaltet:

   - eine Abbildungsvorrichtung (40), die fähig ist, ein radiographisches Bild zu erzeugen, und
   - eine Anzeigevorrichtung (160) zum Anzeigen eines radiographischen Bilds, das durch die Abbildungsvorrichtung erzeugt wird und die Bewegung des Objekts verdeutlicht.

11. Röntgenstrahlenabbildungssystem nach einem der Ansprüche 5 bis 10, wobei die Quelle und die Abbildungsvorrichtung durch einen Bügel verbunden sind.

12. Röntgenstrahlenabbildungssystem nach einem der Ansprüche 5 bis 11, wobei der Kollimator (20) mit Bezug auf die Quelle aus seiner Drehachse bewegbar ist.

13. Verfahren zum Kalibrieren eines Röntgenstrahlenabbildungssystems nach einem der Ansprüche 5 bis 12, wobei das Element eine Abbildungsvorrichtung (40) ist, die mit mindestens 4 Röntgenstrahlendetektoren ausgerüstet ist, beinhaltend das Bestimmen der Lage der Abbildungsvorrichtung mit Bezug auf die Quelle oder mit Bezug auf ein Bezugssystem, das mit einem Raum, in dem sich das Abbildungssystem befindet, verbunden ist, wobei das Bestimmen der Lage der Abbildungsvorrichtung ein Ermitteln, durch eine mit der Abbildungsvorrichtung gekoppelte Verarbeitungseinheit, von mindestens 4 unterschiedlichen Geraden beinhaltet, die jeweils die Quelle und einen Detektor der mindestens 4 Detektoren der Abbildungsvorrichtung verbinden.


**Claims**

1. X-ray imaging device including:

   a collimator formed of a support (20), said carrier being a plate in the form of a disc or portion of a disc,
   the support being capable of being rotationally moveable around itself and with respect to an axis (Δ) of rotation passing through a plane of the support called "main plane of the support",
   the support including a region (22) which is opaque to X-rays, at least one first slot (23) and at least one second slot (24) passing through said opaque region, the first slot and the second slot being transparent to X-rays, the first slot and the second slot each extending in a given plane parallel to the main plane of the support and respectively in a first direction passing through the axis of rotation and in a second direction which is not secant to the axis of rotation, such that when an X-ray source is positioned to provide X-rays, two planes of rotating X-rays are generated downstream of said slots.

2. Device according to claim 1, in which the first slot and the second slot have different widths.

3. Device according to one of claims 1 or 2, in which the support (20) further includes a region (21) which is transparent to X-rays and situated in the given plane and juxtaposed with the opaque region (22).

4. Device according to one of claims 1 to 3, in which the first direction and the second direction form between them a non-zero angle.

5. X-ray imaging system including:

   - an X-ray source (10),
   - a means for determining the position of at least one element (40, 180) with respect to a given coordinate system, the element being provided with one or more X-ray sensors (40a, 40b, 40c, 40d, 140), the determination means including:
- a device according to one of claims 1 or 2,
- a processing unit (50) configured to determine at least one intersecting straight line between a first X-ray beam and a second X-ray beam detected by a same X-ray sensor among said sensors, the first beam coming from the X-ray source and having passed through the first slot and the second X-ray beam coming from the X-ray source and having passed through the second slot.

6. X-ray imaging system according to claim 5, including an imager and in which the support (20) of the collimator is provided with a region (21) which is radio-transparent to X-rays juxtaposed with the radio-opaque region, the system being laid out in such a way that, during a rotation of the collimator around itself, the source is during one phase exposed to an imager through the transparent region then during another phase masked from this imager by the opaque region and exposed uniquely through the first slot and the second slot,
the system being **characterised in that** the processing unit is synchronised with the rotation of the collimator (20) such that:

- during said phase the processing unit triggers the acquisition of at least one radiographic image by the imager,
- during said other phase the processing unit estimates the position of said element.

7. Imaging system according to one of claims 5 or 6, in which said element is an imager (40) and in which said X-ray sensors are pixels of the imager or are arranged on a matrix of pixels of the imager.

8. Imaging system according to one of claims 5 to 7, the determination means being configured to determine the position of the imager with respect to the source.

9. X-ray imaging system according to one of claims 5 or 6, in which the element is an object (180) that can be displaced with respect to the X-ray source, such as a surgical tool, to which the X-ray sensor or sensors (140) is or are attached.

10. X-ray imaging system according to claim 9, the system further including:

- an imager (40) capable of producing a radiographic image and
- a display device (160) for displaying a radiographic image produced by the imager and taking into account the displacement of the object.

11. X-ray imaging system according to one of claims 5 to 10, in which the source and the imager are connected by a C-arm.

12. X-ray imaging system according to one of claims 5 to 11, in which the collimator (20) can be displaced with respect to the source outside of its axis of rotation.

13. Method for calibrating an X-ray imaging system according to one of claims 5 to 12, in which said element is an imager (40) provided with at least 4 X-ray sensors, including the determination of the position of the imager with respect to the source or with respect to a reference coordinate system linked to a room in which the imaging system is located, the determination of the position of the imager including an estimation by a processing unit coupled to the imager of at least 4 distinct straight lines each linking the source and a sensor among said at least 4 sensors of the imager.

**Fig. 1**

FiG2A

FiG2B

**Fig. 3**

## Fig. 4A

## Fig. 4B

Fig. 5

Fig. 6

Fig. 7

Fig. 8

Fig. 9

Fig.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2841118
- US 6490475 B
- US 7065393 B

### Littérature non-brevet citée dans la description

- **GAO et al.** Complété solution classification for the perspective-three-point problem. *IEEE Transactions on Pattern Analysis and Machine Intelligence,* 2003, vol. 25 (8), 930-943
- Method for registration of 3D shapes. **BESL et al.** Robotics-DL tentative. International Society for Optics and Photonics, 1992, 586-606
- **AMIRI et al.** A low-cost tracked C-arm (TC-arm) upgrade system for versatile quantitative intraoperative imaging. *International Journal of Computer Assisted Radiology and Surgery,* 2013, 1-17